(19) Europäisches Patentamt / European Patent Office / Office européen des brevets

(11) **EP 4 474 572 A1**

(12) **EUROPEAN PATENT APPLICATION**

(43) Date of publication:
**11.12.2024 Bulletin 2024/50**

(21) Application number: **24180191.9**

(22) Date of filing: **05.06.2024**

(51) International Patent Classification (IPC):
**D21F 7/00** *(2006.01)*     **D21F 7/06** *(2006.01)*
**D21G 1/00** *(2006.01)*

(52) Cooperative Patent Classification (CPC):
**D21F 7/003; D21F 7/06; D21G 1/002**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.06.2023  JP 2023095873**

(71) Applicant: **Yokogawa Electric Corporation Tokyo 180-8750 (JP)**

(72) Inventors:
• **NISHIDA, Kazufumi**
  **Musashino-shi, Tokyo, 180-8750 (JP)**
• **HORIKOSHI, Kumiko**
  **Musashino-shi, Tokyo, 180-8750 (JP)**

(74) Representative: **Winter, Brandl - Partnerschaft mbB**
**Alois-Steinecker-Straße 22**
**85354 Freising (DE)**

(54) **INFORMATION PROVIDING APPARATUS, INFORMATION PROVIDING METHOD, AND INFORMATION PROVIDING PROGRAM**

(57)     An information providing apparatus 10 according to one embodiment includes an acquisition unit 141, a calculation unit 142, and a providing unit (143). The acquisition unit (141) acquires a thickness measurement value of each of portions of paper and a moisture percentage measurement value of each of the portions of the paper before or after a pressing process of pressing each of the portions of the paper with a different pressing force by using a roll based on an index that is provided. The calculation unit 142 calculates an estimated value of a thickness of the paper after a moisture percentage of the paper has changed, based on the thickness measurement value and the moisture percentage measurement value. The providing unit 143 provides the estimated value as the index to an apparatus that performs the pressing process.

FIG.1

EP 4 474 572 A1

**Description**

FIELD

The present invention relates to an information providing apparatus, an information providing method, and information providing program.

BACKGROUND

Conventionally, a technology for equalizing a thickness of a paper web (sheet-like paper) in a paper manufacturing process is known.

For example, Japanese Laid-open Patent Publication No. 2004-277899 describes a technology for adjusting linear pressure in a width direction in a pressing part based on a measured thickness profile of a paper web.

Patent Literature 1: Japanese Laid-open Patent Publication No. 2003-27396

Patent Literature 3: Japanese Laid-open Patent Publication No. 2013-108859

However, in the conventional technology, in some cases, it is difficult to equalize a thickness of paper in a paper manufacturing process with high accuracy.

For example, the thickness of the paper changes with a change in an amount of moisture contained in the paper. The amount of moisture in the paper changes in processes subsequent to the process of adjusting the thickness and changes even after manufacturing. In contrast, in the conventional technology, a change of the thickness of paper due to a change in the amount of moisture is not fully taken into account.

According to one aspect, an object is to equalize a thickness of paper in a paper manufacturing process with high accuracy.

SUMMARY

According to an aspect of the embodiments, an information providing apparatus includes an acquisition unit that acquires a thickness measurement value of each of portions of paper and a moisture percentage measurement value of each of the portions of the paper before or after a pressing process of pressing each of the portions of the paper with a different pressing force by using a roll based on an index that is provided; a calculation unit that calculates an estimated value of a thickness of the paper after a moisture percentage of the paper has changed, based on the thickness measurement value and the moisture percentage measurement value; and a providing unit that provides the estimated value as the index to an apparatus that performs the pressing process.

According to an aspect of the embodiments, an information providing method implemented by a computer, the information providing method includes acquiring a thickness measurement value of each of portions of paper and a moisture percentage measurement value of each of the portions of the paper before or after a pressing process of pressing each of the portions of the paper with a different pressing force by using a roll based on an index that is provided; calculating an estimated value of a thickness of the paper after a moisture percentage of the paper has changed, based on the thickness measurement value and the moisture percentage measurement value; and providing the estimated value as the index to an apparatus that performs the pressing process.

According to an aspect of the embodiments, an information providing program causing a computer to execute processing includes acquiring a thickness measurement value of each of portions of paper and a moisture percentage measurement value of each of the portions of the paper before or after a pressing process of pressing each of the portions of the paper with a different pressing force by using a roll based on an index that is provided; calculating an estimated value of a thickness of the paper after a moisture percentage of the paper has changed, based on the thickness measurement value and the moisture percentage measurement value; and providing the estimated value as the index to an apparatus that performs the pressing process.

BRIEF DESCRIPTION OF THE DRAWINGS

FIG. 1 is a diagram illustrating a configuration example of an adjustment system according to one embodiment;
FIG. 2 is a diagram illustrating a configuration example of an information providing apparatus according to one embodiment;
FIG. 3 is a diagram for explaining a synthesized thickness profile;
FIG. 4 is a flowchart illustrating the flow of a process performed by the information providing apparatus according to one embodiment; and

FIG. 5 is a diagram for explaining a hardware configuration example.

DESCRIPTION OF EMBODIMENTS

Embodiments of an information providing apparatus, an information providing method, and an information providing program according to the present disclosure will be described in detail below based on the drawings. Meanwhile, the present invention is not limited by the embodiments described below. Further, the same components are denoted by the same reference symbols, and repeated explanation will be omitted appropriately. Furthermore, the embodiments may be combined appropriately as long as no contradiction is derived.

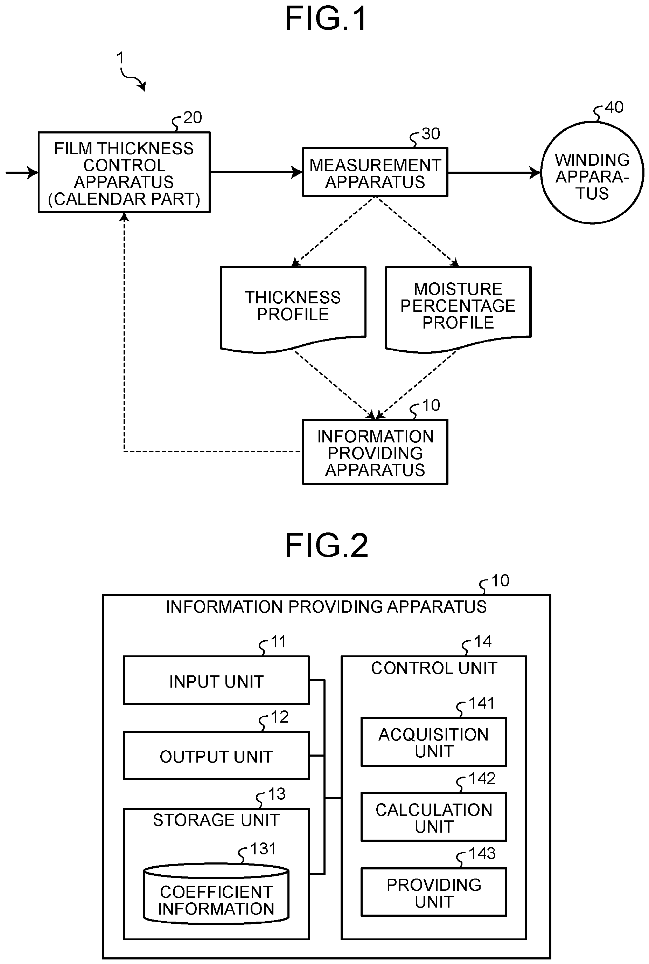

A configuration of an adjustment system including an information providing apparatus will be described with reference to FIG. 1. The adjustment system includes at least a part of a paper manufacturing line and the information providing apparatus.

As illustrated in FIG. 1, an adjustment system 1 includes an information providing apparatus 10, a film thickness control apparatus 20, a measurement apparatus 30, and a winding apparatus 40. Meanwhile, bold arrows in FIG. 1 indicate a flow of paper to be manufactured. Further, dashed arrows indicate a flow of data or a signal.

The paper manufacturing line includes a wire part, a press part, a dryer part, a calendar part, a winding process, and the like. Paper in a certain state in which some amount of moisture is removed is supplied to the calendar part. In the calendar part, pressure is applied to the supplied paper by a roll to give a shine. Meanwhile, the film thickness control apparatus 20 and the winding apparatus 40 may be a part of a paper machine.

The film thickness control apparatus 20 performs a process in the calendar part. Specifically, the film thickness control apparatus 20 controls a pressing force to adjust a paper thickness. The film thickness control apparatus 20 changes a pressing force at each position in a paper width direction (perpendicular to a paper conveying direction and parallel to the paper). For example, the film thickness control apparatus 20 may change the pressing force by changing a roll diameter by locally heating the calendar roll, or may change the pressing force by a large number of actuators that are arranged inside an elastically deformable roll.

The measurement apparatus 30 measures a distribution of a paper thickness in a width direction (thickness profile) and a distribution of a moisture percentage in the width direction (moisture percentage profile) of the paper for which the thickness is adjusted in the calendar part. The measurement apparatus 30 includes a sensor that performs scanning in the paper width direction. The sensor measures the thickness and the moisture percentage.

The information providing apparatus 10 calculates an index for adjusting the paper thickness based on the thickness profile and the moisture percentage profile. The information providing apparatus 10 provides the calculated index to the film thickness control apparatus 20.

For example, the index is an estimated value of a thickness in each of portions in the paper width direction, in which the moisture percentage is taken into account. The paper thickness that is measured by the measurement apparatus 30 may further change with a change in the moisture percentage in the subsequent process. Therefore, for example, even if the paper thickness is uniform immediately after the calendar part, a final paper thickness is not always uniform.

Therefore, the film thickness control apparatus 20 adjusts the paper thickness based on the index that is calculated by taking into account the paper thickness and the moisture percentage, rather than based on a measurement value of the paper thickness. With this configuration, it is possible to more accurately equalize a thickness of finally completed paper. Meanwhile, a specific method of calculating the index will be described later.

A configuration of the information providing apparatus 10 will be described below with reference to FIG. 2. FIG. 2 is a diagram illustrating a configuration example of the information providing apparatus according to one embodiment. As illustrated in FIG. 2, the information providing apparatus 10 includes an input unit 11, an output unit 12, a storage unit 13, and a control unit 14.

The input unit 11 is an interface for inputting data. The input unit 11 receives input of data from the measurement apparatus 30. The output unit 12 is an interface for outputting data. The output unit 12 outputs data to the film thickness control apparatus 20.

The storage unit 13 stores therein various kinds of data, various programs executed by the control unit 14, and the like. For example, the storage unit 13 is a storage device, such as a memory or a hard disk. The storage unit 13 stores therein various kinds of data that are generated by a process performed by the information providing apparatus 10, such as data that is obtained while the control unit 14 performs various kinds of processing and processing results that are obtained by execution of various kinds of processing. The storage unit 13 stores therein coefficient information 131. The coefficient information 131 is a value of a coefficient that is used for calculation of the index.

The control unit 14 is a processing unit that controls the entire information providing apparatus 10. The control unit 14 includes an acquisition unit 141, a calculation unit 142, and a providing unit 143.

The acquisition unit 141 acquires the thickness profile and the moisture percentage profile from the measurement apparatus 30.

The calculation unit 142 calculates the index based on the thickness profile and the moisture percentage profile.

The calculation unit 142 calculates a synthesized thickness profile as the index. The synthesized thickness profile is an estimated value of an oven-dried paper thickness or an air-dried paper thickness.

The oven-dried paper thickness is a paper thickness that is obtained when manufactured paper is completely dried (the moisture percentage is 0%). Further, the air-dried paper thickness is a paper thickness that is obtained when a portion of manufactured paper in which the moisture percentage is high is naturally dried and the moisture percentage is equalized.

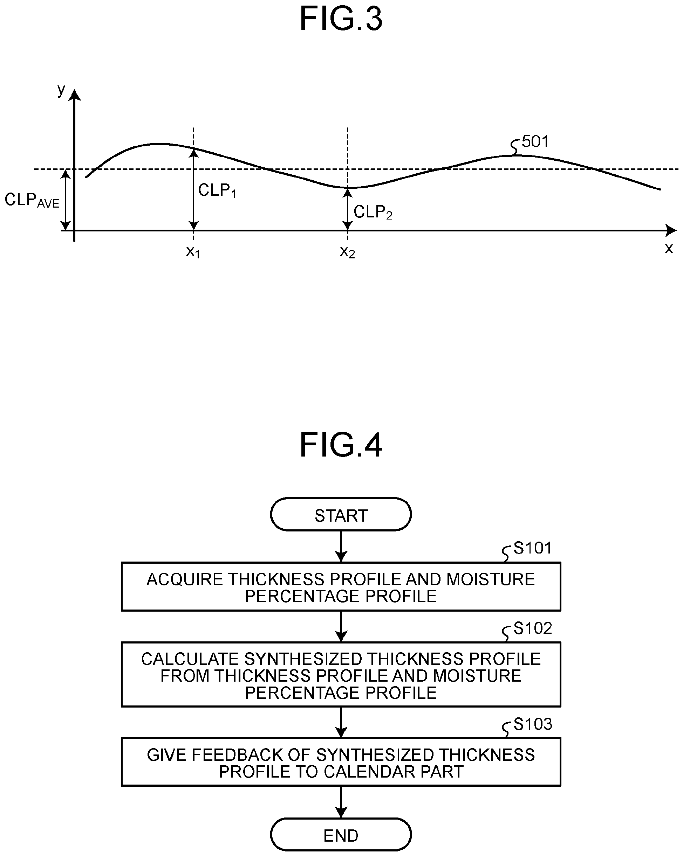

FIG. 3 is a diagram for explaining the synthesized thickness profile. An x axis is an axis that represents a position in the paper width direction. A y axis is an axis that represents a paper thickness. A curved line 501 is a curved line that indicates the paper thickness.

A value of x indicates a portion of the paper as the position in the paper width direction. The thickness profile that is acquired by the acquisition unit 141 is a paper thickness corresponding to each of the values of x. Further, the moisture percentage profile that is acquired by the acquisition unit 141 is a paper moisture percentage corresponding to each of the values of x.

For example, the thickness profile indicates that a thickness measurement value at a position of $x = x_i$ is $CLP_1$. Further, for example, the thickness profile indicates that a thickness measurement value at a position of $x = x_2$ is $CLP_2$. Furthermore, $CLP_{AVE}$ indicates an average value of the thickness measurement values at all of the positions.

Although not illustrated in FIG. 3, the moisture percentage profile indicates a moisture percentage measurement value at each of the positions. For example, the moisture percentage profile indicates that a moisture percentage measurement value at a position of $x = x_i$ is $MP_1$. Further, for example, the moisture percentage profile indicates that a moisture percentage measurement value at a position of $x = x_2$ is $MP_2$.

For example, the calculation unit 142 calculates the synthesized thickness profile (the oven-dried paper thickness or the air-dried paper thickness) at each of the positions in the paper width direction by at least one of Expressions (1) to (7) below. Here, CLP and MP respectively indicate the thickness measurement value and the moisture percentage measurement value at each of the positions.

$\alpha$ and $\beta$ are conversion coefficients that are determined in advance and take positive values. $\alpha$ and $\beta$ are included in the coefficient information 131. $\Delta MP$ is a difference between a moisture percentage fixed value that is set in advance or an average value ($MP_{AVE}$) of the moisture percentage measurement values at all of the positions and the moisture percentage measurement value MP. Further, a function f(z) is a function that represents a film thickness change with respect to a moisture percentage z. For example, a paper thickness at the moisture percentage of z is represented such that the oven-dried paper thickness + f(z).

As indicated by Expression (1), the calculation unit 142 is able to calculate the oven-dried paper thickness by subtracting, from the thickness measurement value CLP, a value that is obtained by multiplying the conversion coefficient $\alpha$ and the moisture percentage measurement value MP.

$$\text{Oven-dried paper thickness} = CLP - \alpha * MP \qquad (1)$$

As indicated by Expression (2), the calculation unit 142 is able to calculate the oven-dried paper thickness by subtracting, from the thickness measurement value CLP, a value that is obtained by multiplying the conversion coefficient $\beta$, the moisture percentage measurement value MP, and the thickness measurement value CLP.

$$\text{Oven-dried paper thickness} = CLP - \beta * MP * CLP \qquad (2)$$

As indicated by Expression (3), the calculation unit 142 is able to calculate the oven-dried paper thickness by subtracting, from the thickness measurement value CLP, a value that is obtained by multiplying the conversion coefficient $\beta$, the moisture percentage measurement value MP, and the average value $CLP_{AVE}$ of the thickness measurement values.

$$\text{Oven-dried paper thickness} = CLP - \beta * MP * CLP_{AVE} \qquad (3)$$

As indicated by Expression (4), the calculation unit 142 is able to calculate the air-dried paper thickness by subtracting, from the thickness measurement value CLP, a value that is obtained by multiplying the conversion coefficient $\alpha$ and $\Delta MP$.

$$\text{Air-dried paper thickness} = CLP - \alpha * (\Delta MP) \qquad (4)$$

As indicated by Expression (5), the calculation unit 142 is able to calculate the air-dried paper thickness by subtracting, from the thickness measurement value CLP, a value that is obtained by multiplying the conversion coefficient β, ΔMP, and the thickness measurement value CLP.

$$\text{Air-dried paper thickness} = \text{CLP} - \beta * (\Delta\text{MP}) * \text{CLP} \quad (5)$$

As indicated by Expression (6),the calculation unit 142 is able to calculate the air-dried paper thickness by subtracting, from the thickness measurement value CLP, a value that is obtained by multiplying the conversion coefficient β, ΔMP, and the average value $\text{CLP}_{AVE}$ of the thickness measurement values.

$$\text{Air-dried paper thickness} = \text{CLP} - \beta * (\Delta\text{MP}) * \text{CLP}_{AVE}$$

$$(6)$$

As indicated by Expression (7), the calculation unit 142 is able to calculate the air-dried paper thickness by subtracting, from the thickness measurement value CLP, a difference between a film thickness change that occurs when the moisture percentage is the measurement value MP and a film thickness change that occurs when the moisture percentage is the air-dried moisture percentage (for example, 6%). The air-dried moisture percentage is set in advance.

$$\text{Air-dried paper thickness} = \text{CLP} - \{f(\text{MP}) - f(\text{air-dried moisture percentage})\}$$

$$(7)$$

Expressions (2), (3), (5), and (6) are expressions in which a change of the paper thickness due to a change of the moisture percentage is proportional to not only the moisture percentage, but also the paper thickness itself (CLP or $\text{CLP}_{AVE}$ in the second term of the right side of each of Expressions). With this configuration, it is possible to keep the conversion coefficient within a certain range for any brand with a different paper thickness, so that it is possible to easily manage brands.

Furthermore, in Expressions (2) and (5), the conversion coefficient is multiplied by the thickness measurement value at each of the positions. In contrast, it is possible to assume that unevenness of the paper thickness is adequately small with respect to a correction amount, and therefore, CLP that is multiplied to the conversion coefficient may be replaced with the average value $\text{CLP}_{AVE}$ as indicated by Expressions (3) and (6). Moreover, the value that is multiplied to the conversion coefficient is not limited to CLP and $\text{CLP}_{AVE}$, but may be any amount, such as a basis weight or an oven-dried basis weight, that is proportional to a swelling amount of paper. Meanwhile, when the basis weight is used as a value that is multiplied to the conversion coefficient, moisture percentage ×basis weight indicates an amount of moisture that is present in the paper.

The measurement apparatus 30 may directly measure and output the amount of moisture that is present in the paper, instead of the moisture percentage. The information providing apparatus 10 is able to calculate the synthesized thickness profile by using the amount of moisture instead of the moisture percentage. In this case, Expressions (1) and (4) effectively function by replacing the moisture percentage with the amount of moisture. Further, in this case, it is possible to keep a conversion coefficient for any brand with a different paper thickness in a certain range without multiplying the thickness or the basis weight as in Expressions (2), (3), (5), and (6), so that it is possible to more easily manage brands, which is an advantage.

For example, the calculation unit 142 is able to calculate the oven-dried paper thickness by Expression (8) in which the moisture percentage MP used in Expression (1) is replaced with a moisture amount MW.

$$\text{Oven-dried paper thickness} = \text{CLP} - \alpha * \text{MW} \quad (8)$$

Furthermore, for example, the calculation unit 142 is able to calculate the air-dried paper thickness by Expression (9) in which ΔMP used in Expression (4) is replaced with ΔMW. However, ΔMW is a difference between a fixed value of the amount of moisture that is set in advance or an average value ($\text{MW}_{AVE}$) of the measurement values of the amounts of moisture at all of the positions and a moisture amount measurement value MW.

$$\text{Air-dried paper thickness} = CLP - \alpha * (\Delta MW) \qquad (9)$$

Moreover, the measurement apparatus 30 is able to measure the basis weight. In this case, the acquisition unit 141 further acquires a distribution of basis weights of a plurality of portions of the paper, that is, a basis weight profile, from the measurement apparatus 30. In this case, the calculation unit 142 calculates a synthesized profile by using the basis weight profile.

For example, the calculation unit 142 is able to calculate the oven-dried paper thickness by Expression (10) or Expression (11) in which CLP in the second term of the right side of each of Expressions (2) and (3) is replaced with a basis weight measurement value BW.

$$\text{Oven-dried paper thickness} = CLP - \beta * MP * BW \quad (10)$$

$$\text{Oven-dried paper thickness} = CLP - \beta * MP * BW_{AVE} \qquad (11)$$

Furthermore, for example, the calculation unit 142 is able to calculate the air-dried paper thickness by Expression (12) or Expression (13) in which $CLP_{AVE}$ in the second term of the right side of each of Expressions (5) and (6) is replaced with an average value $BW_{AVE}$ of the basis weight measurement values.

$$\text{Air-dried paper thickness} = CLP - \beta * (\Delta MP) * BW \quad (12)$$

$$\text{Air-dried paper thickness} = CLP - \beta * (\Delta MP) * BW_{AVE}$$

$$(13)$$

The providing unit 143 provides the synthesized paper thickness to the film thickness control apparatus 20. Specifically, the providing unit 143 gives a feedback of the synthesized thickness profile to the film thickness control apparatus 20 based on the thickness profile and the moisture percentage profile that are obtained as a result of processing that is performed by the film thickness control apparatus 20 in the calendar part. With this configuration, the adjustment system 1 realizes feedback control.

The film thickness control apparatus 20 controls a paper thickness along the paper width direction based on the synthesized thickness profile. Accordingly, even when the paper is dried after manufacturing, the paper thickness with deduction of an influence of moisture is maintained uniform, so that it is possible to expect improvement in paper quality.

For example, it is assumed that, when the information providing apparatus 10 calculates the synthesized thickness profile by using Expression(4) with respect to the paper thickness (CLP) of 60 um while $\alpha$ = 0.8, it is possible to correct a thickness change due to a moisture percentage change. In this case, assuming that $\beta = \alpha / 60 = 0.8 / 60 = 0.0133$, it is possible to assume that the information providing apparatus 10 is able to similarly correct a thickness change due to a moisture percentage change by calculating the synthesized thickness profile by using Expression (5) or Expression (6). In this manner, according to the information providing apparatus 10, when a brand with a different film thickness is to be manufactured, it is possible to use, as a conversion coefficient, a certain value that is close to a conversion coefficient with which correction is successful.

A flow of a process performed by the information providing apparatus 10 will be described below with reference to FIG. 4. FIG. 4 is a flowchart illustrating the flow of the process performed by the information providing apparatus according to one embodiment.

As illustrated in FIG. 4, first, the information providing apparatus 10 acquires the thickness profile and the moisture percentage profile from the measurement apparatus 30 (Step S101). Subsequently, the information providing apparatus 10 calculates the synthesized thickness profile from the thickness profile and the moisture percentage profile (Step S102). Furthermore, the information providing apparatus 10 gives a feedback of the synthesized thickness profile to the calendar part, that is, the film thickness control apparatus 20 (Step S103).

The information providing apparatus 10 is able to further improve accuracy of paper thickness adjustment based on feedback control by repeating the processes from Step S101 to Step S103.

Effects

As described above, the information providing apparatus 10 includes the acquisition unit 141, the calculation unit 142, and the providing unit 143. The acquisition unit 141 acquires a thickness measurement value of each of portions of paper and a moisture percentage measurement value of each of the portions of the paper before or after a pressing process of pressing each of the portions of the paper with a different pressing force by using a roll based on an index that is provided. The calculation unit 142 calculates an estimated value of a thickness of the paper after a moisture percentage of the paper has changed, based on the thickness measurement value and the moisture percentage measurement value. The providing unit 143 provides the estimated value as the index to an apparatus that performs the pressing process.

The pressing process of pressing each of the portions with a different pressing force by using the roll based on the provided index is the calendar part and is performed by the film thickness control apparatus 20. Further, the measurement apparatus 30 may measure the paper thickness and the paper moisture percentage after the calendar part as described above, or may measure the paper thickness and the paper moisture percentage before the calendar part. The estimated value of the paper thickness after the paper moisture percentage has changed is the oven-dried paper thickness or the air-dried paper thickness that is explained above with reference to Expression (1) to Expression (7).

As described above, the information providing apparatus 10 is able to calculate the index by taking into account not only the paper thickness measurement value, but also the moisture percentage measurement value. With this configuration, it is possible to improve accuracy of paper thickness adjustment based on feedback control. As a result, according to one embodiment, it is possible to accurately equalize the paper thickness in a paper manufacturing process.

Here, if the paper thickness is uneven, the paper may be unsuccessfully conveyed at the time of printing, printing may fail, printing quality may become unstable, or a product value of the paper may be degraded. According to one embodiment as described above, it is possible to prevent situations as described above.

Furthermore, the film thickness control apparatus 20 adjusts, in the calendar part, the thickness in the paper width direction such that the air-dried paper thickness that is provided as the index becomes even. With this adjustment, the thickness profile of the paper in the air-dried state becomes uniform.

In this manner, according to one embodiment, unevenness of a moisture percentage distribution after manufacturing is eliminated, so that even when a thickness of manufactured paper is partly changed, a final paper thickness becomes even. With this configuration, thickness unevenness due to moisture absorption and moisture desorption of manufactured paper is reduced, so that it is possible to improve quality of a paper product.

With the uniform thickness of the manufactured paper, it is possible to prevent situations in which ink paint unevenness occurs, dimensions of a processed product are uneven, and the like. Furthermore, according to one embodiment, it is possible to prevent unevenness of a wound paper roll and prevent a quality defect, such as wrinkles in paper. Moreover, according to one embodiment, it is possible to prevent unevenness of the paper roll, so that it is possible to reduce a paper jam when paper is mounted on a machine, reduce instability of the machine, reduce a damage, or the like. Furthermore, according to one embodiment, it is possible to reduce energy that is needed to equalize the moisture percentage profile, so that it is possible to contribute to reduction of resources and environmental protection.

Modification

Embodiments may be appropriately modified as described below.

For example, a portion that controls a paper thickness in the adjustment system 1 may be other than the calendar part. Furthermore, the calendar part may perform a process of adjusting the paper moisture percentage before a process of controlling the paper thickness. In this manner, if the moisture percentage is set so as to fall within a certain range in advance by adjustment of the moisture percentage, the information providing apparatus 10 is able to calculate the oven-dried paper thickness and the air-dried paper thickness with high efficiency and high accuracy.

Moreover, in the calendar part, it may be possible to simultaneously perform control of equalizing the thickness and control of equalizing the moisture percentage. With this configuration, an amount of adjustment in the control of equalizing the thickness becomes more constant, so that it is possible to uniformly achieve the effect in which a paper surface is made smooth by the calendar part.

Furthermore, in the control of equalizing the moisture percentage profile, it may be possible to perform control of allowing a certain amount of deviation from target evenness. With this configuration, it is possible to maintain a certain level of the effect in which the paper surface is made smooth by the calendar part, and it is possible to reduce energy that is input for adjustment of the moisture percentage profile.

Moreover, the control of equalizing the paper thickness may be performed by adjusting a moisture percentage in the width direction while maintaining a constant linear pressure of the roll for pressing in the calendar part. In this case, it is possible to equalize the final paper thickness while maintaining a constant effect in which the paper surface is made smooth by the calendar part.

Furthermore, the moisture percentage may be adjusted such that the moisture percentage is reduced at a paper edge without being equalized in the paper width direction. When the moisture percentage is adjusted so as to be reduced at the paper edge, an actual paper thickness for equalizing an air-dried film thickness profile is not an even profile, but a profile in which the edge is thin. Paper with a paper thickness distribution as described above can easily emit air at the time of winding, so that it may be possible to reduce meandering of the paper at the time of winding and it may be possible to easily handle the paper.

The information providing apparatus 10 may perform a calculation for performing control such that deviation from a measured film thickness falls within a certain range when calculating the air-dried paper thickness. Here, the thickness at the time of paper winding is close to the measured film thickness, so that if a correction amount is too large, the paper is wound with a deviated film thicknesses, so that wrinkles may occur and it may become difficult to wind the paper in a good manner. In contrast, if the correction amount is limited, a winding posture is stabilized, so that it is possible to prevent suspension of operation due to a winding failure and it is possible to continue the operation.

System

The processing procedures, control procedures, specific names, and information including various kinds of data and parameters illustrated in the above-described document and drawings may be arbitrarily changed unless otherwise specified.

Furthermore, the components of the apparatuses illustrated in the drawings are functionally conceptual and do not necessarily have to be physically configured in the manner illustrated in the drawings. In other words, specific forms of distribution and integration of the apparatuses are not limited to those illustrated in the drawings. Namely, all or part of the apparatuses may be functionally or physically distributed or integrated in arbitrary units depending on various loads or use conditions.

Moreover, for each processing function performed by each apparatus, all or any part of the processing function may be implemented by a CPU and a program analyzed and executed by the CPU or may be implemented as hardware by wired logic.

Hardware

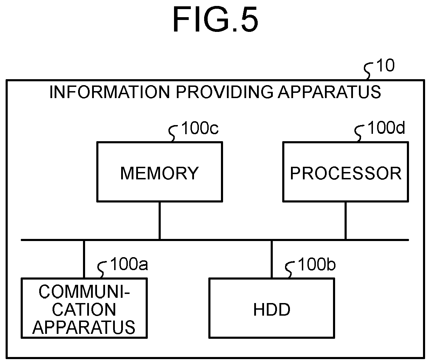

A hardware configuration example of the information providing apparatus 10 will be described below. FIG. 5 is a diagram for explaining the hardware configuration examples. As illustrated in FIG. 5, the information providing apparatus 10 includes a communication apparatus 100a, a Hard Disk Drive (HDD) 100b, a memory 100c, and a processor 100d. Further, all of the units illustrated in FIG. 5 are connected to one another by a bus or the like.

The communication apparatus 100a is a network interface card or the like, and performs communication with a different server. The HDD 100b stores therein a program and a DB for implementing the functions as illustrated in FIG. 2.

The processor 100d reads a program for performing the same processes as those of each of the processing units as illustrated in FIG. 2 from the HDD 100b or the like, loads the program onto the memory 100c, and operates a process for implementing each of the functions explained above with reference to FIG. 2 or the like. For example, the process implements the same functions as those of each of the processing units included in the information providing apparatus 10. Specifically, the processor 100d performs a process for implementing the same processes as those of the acquisition unit 141, the calculation unit 142 and the providing unit 143.

In this manner, the information providing apparatus 10 functions as an information providing apparatus that implements an information providing method by reading and executing a program. Furthermore, the information providing apparatus 10 is able to implement the same functions as those of the embodiment as described above by causing a medium reading apparatus to read the above-described program from a recording medium and executing the read program. Meanwhile, the program described in the different embodiment need not always be executed by the information providing apparatus 10. For example, the present invention may be applied in the same manner in a case in which a different computer or a different server executes the program or a case in which the different computer and the different server execute the program in a cooperative manner.

The program may be distributed via a network, such as the Internet. Further, the program may be recorded in a computer readable recording medium, such as a hard disk, a flexibly disk (FD), a compact disc-read only memory (CD-ROM), a MagnetoOptical disk (MO), or a Digital Versatile Disk (DVD), and may be executed by being read from the recording medium by a computer.

According to one embodiment of the present invention, it is possible to equalize a thickness of paper in a paper manufacturing process with high accuracy.

Although the invention has been described with respect to specific embodiments for a complete and clear disclosure, the appended claims are not to be thus limited but are to be construed as embodying all modifications and alternative constructions that may occur to one skilled in the art that fairly fall within the basic teaching herein set forth.

**Claims**

1. An information providing apparatus (10) comprising:

   an acquisition unit (141) that acquires a thickness measurement value of each of portions of paper and a moisture percentage measurement value of each of the portions of the paper before or after a pressing process of pressing each of the portions of the paper with a different pressing force by using a roll based on an index that is provided; a calculation unit (142) that calculates an estimated value of a thickness of the paper after a moisture percentage of the paper has changed, based on the thickness measurement value and the moisture percentage measurement value; and
   a providing unit (143) that provides the estimated value as the index to an apparatus that performs the pressing process.

2. The information providing apparatus (10) according to claim 1, wherein the calculation unit (142) calculates the index by using the thickness measurement value of each of the portions of the paper and a difference between the moisture percentage measurement value and one of a moisture percentage fixed value that is set in advance and an average value of the moisture percentage measurement values.

3. The information providing apparatus (10) according to claim 1, the calculation unit (142) calculates the index by subtracting, from the thickness measurement value, a value that is obtained by multiplying a coefficient, the moisture percentage measurement value, and the thickness measurement value.

4. The information providing apparatus (10) according to claim 1, the calculation unit (142) calculates the index by subtracting, from the thickness measurement value, a value that is obtained by multiplying a coefficient, the moisture percentage measurement value, and an average value of the thickness measurement values.

5. The information providing apparatus (10) according to claim 1, the calculation unit (142) calculates the index by subtracting, from the thickness measurement value, a value that is obtained by multiplying a coefficient and a difference between the moisture percentage measurement value and one of a moisture percentage fixed value that is set in advance and an average value of the moisture percentage measurement values.

6. The information providing apparatus (10) according to claim 1, the calculation unit (142) calculates the index by subtracting, from the thickness measurement value, a value that is obtained by multiplying a coefficient, the thickness measurement value, and a difference between the moisture percentage measurement value and one of a moisture percentage fixed value that is set in advance and an average value of the moisture percentage measurement values.

7. The information providing apparatus (10) according to claim 1, wherein the calculation unit (142) calculates the index by subtracting, from the thickness measurement value, a value that is obtained by multiplying a coefficient, an average value of the thickness measurement values, and a difference between the moisture percentage measurement value and one of a moisture percentage fixed value that is set in advance and an average value of the moisture percentage measurement values.

8. The information providing apparatus (10) according to claim 1, wherein the calculation unit (142) calculates the index by subtracting, from the thickness measurement value, a difference between a film thickness change with respect to the moisture percentage measurement value and a film thickness change with respect to an air-dried moisture percentage.

9. The information providing apparatus (10) according to claim 1, wherein

   the acquisition unit (141) further acquires a basis weight measurement value of each of the portions of the paper, and
   the calculation unit (142) calculates the index by subtracting, from the thickness measurement value, a value that is obtained by multiplying a coefficient, the moisture percentage measurement value, and the basis weight measurement value.

10. The information providing apparatus (10) according to claim 1, wherein

   the acquisition unit (141) further acquires a basis weight measurement value of each of the portions of the

9

paper, and
the calculation unit (142) calculates the index by subtracting, from the thickness measurement value, a value that is obtained by multiplying a coefficient, the basis weight measurement value, and a difference between the moisture percentage measurement value and one of a moisture percentage fixed value that is set in advance and an average value of the moisture percentage measurement values.

11. An information providing method implemented by a computer, the information providing method comprising:

acquiring a thickness measurement value of each of portions of paper and a moisture percentage measurement value of each of the portions of the paper before or after a pressing process of pressing each of the portions of the paper with a different pressing force by using a roll based on an index that is provided;
calculating an estimated value of a thickness of the paper after a moisture percentage of the paper has changed, based on the thickness measurement value and the moisture percentage measurement value; and
providing the estimated value as the index to an apparatus that performs the pressing process.

12. An information providing program causing a computer to execute processing comprising:

acquiring a thickness measurement value of each of portions of paper and a moisture percentage measurement value of each of the portions of the paper before or after a pressing process of pressing each of the portions of the paper with a different pressing force by using a roll based on an index that is provided;
calculating an estimated value of a thickness of the paper after a moisture percentage of the paper has changed, based on the thickness measurement value and the moisture percentage measurement value; and
providing the estimated value as the index to an apparatus that performs the pressing process.

# FIG.1

1

| FILM THICKNESS CONTROL APPARATUS (CALENDAR PART) | ~20 |
|---|---|

| MEASUREMENT APPARATUS | ~30 |

| WINDING APPARA-TUS | ~40 |

| THICKNESS PROFILE |

| MOISTURE PERCENTAGE PROFILE |

| INFORMATION PROVIDING APPARATUS | ~10 |

# FIG.2

## INFORMATION PROVIDING APPARATUS ~10

| INPUT UNIT | ~11 |

| OUTPUT UNIT | ~12 |

| STORAGE UNIT | ~13 |

| COEFFICIENT INFORMATION | ~131 |

### CONTROL UNIT ~14

| ACQUISITION UNIT | ~141 |

| CALCULATION UNIT | ~142 |

| PROVIDING UNIT | ~143 |

# FIG.3

# FIG.4

```
        ┌─────────────┐
        │    START    │
        └──────┬──────┘
               │
               ▼              ⌐S101
┌──────────────────────────────────────┐
│  ACQUIRE THICKNESS PROFILE AND         │
│       MOISTURE PERCENTAGE PROFILE       │
└──────────────────┬─────────────────────┘
                   │
                   ▼          ⌐S102
┌──────────────────────────────────────┐
│  CALCULATE SYNTHESIZED THICKNESS        │
│  PROFILE FROM THICKNESS PROFILE AND     │
│     MOISTURE PERCENTAGE PROFILE         │
└──────────────────┬─────────────────────┘
                   │
                   ▼          ⌐S103
┌──────────────────────────────────────┐
│  GIVE FEEDBACK OF SYNTHESIZED           │
│  THICKNESS PROFILE TO CALENDAR PART     │
└──────────────────┬─────────────────────┘
                   │
                   ▼
        ┌─────────────┐
        │     END     │
        └─────────────┘
```

# FIG.5

INFORMATION PROVIDING APPARATUS — 10

MEMORY — 100c

PROCESSOR — 100d

COMMUNI-CATION APPARATUS — 100a

HDD — 100b

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | EP 0 298 057 A2 (VALMET PAPER MACHINERY INC [FI]) 4 January 1989 (1989-01-04)<br>* column 1, line 21 - line 58 *<br>* column 9, line 50 - column 10, line 18 *<br>* figures * | 1-8,11, 12<br><br>9,10 | INV.<br>D21F7/00<br>D21F7/06<br>D21G1/00 |
| X<br><br>Y | DE 30 07 452 A1 (BRUDERHAUS MASCHINEN GMBH [DE]) 3 September 1981 (1981-09-03)<br>* page 9, line 1 - line 32 *<br>* figures * | 1-8,11, 12<br><br>9,10 | |
| X | TUOMISTO M V ET AL: "SUPERCALENDERS GO HIGH TECH WITH RAPID ADVANCES IN AUTOMATION AND QUALITY CONTROL SYSTEMS", TAPPI JOURNAL, TECHNICAL ASSOCIATION OF THE PULP & PAPER INDUSTRY, ATLANTA US, vol. 74, no. 2, 1 February 1991 (1991-02-01), pages 93-101, XP000145570, ISSN: 0734-1415<br>* Supercalender operating parameters *<br>* Zone controlled rolls *<br>* On-line quality measurement * | 1-12 | |
| Y | WO 97/10383 A1 (VALMET KARLSTAD AB [SE]) 20 March 1997 (1997-03-20)<br>* page 1, line 5 - line 15 *<br>* page 2, line 25 - page 4, line 16 * | 9,10 | TECHNICAL FIELDS SEARCHED (IPC)<br><br>D21F<br>D21G |

The present search report has been drawn up for all claims

| Place of search | Date of completion of the search | Examiner |
|---|---|---|
| Munich | 19 September 2024 | Arndt, Markus |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ..............................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 18 0191

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2024

| Patent document cited in search report | | Publication date | Patent family member(s) | | Publication date |
|---|---|---|---|---|---|
| EP 0298057 | A2 | 04-01-1989 | AT | E70098 T1 | 15-12-1991 |
| | | | AT | E110809 T1 | 15-09-1994 |
| | | | CA | 1282619 C | 09-04-1991 |
| | | | DE | 3851340 T2 | 19-01-1995 |
| | | | EP | 0298057 A2 | 04-01-1989 |
| | | | EP | 0449390 A2 | 02-10-1991 |
| | | | ES | 2059038 T3 | 01-11-1994 |
| | | | JP | 2788009 B2 | 20-08-1998 |
| | | | JP | S63295788 A | 02-12-1988 |
| | | | US | 4791863 A | 20-12-1988 |
| DE 3007452 | A1 | 03-09-1981 | AT | E12667 T1 | 15-04-1985 |
| | | | DE | 3007452 A1 | 03-09-1981 |
| | | | EP | 0035110 A1 | 09-09-1981 |
| | | | FI | 69595 B | 29-11-1985 |
| | | | JP | S5930837 B2 | 28-07-1984 |
| | | | JP | S56136320 A | 24-10-1981 |
| | | | US | 4380954 A | 26-04-1983 |
| | | | YU | 18981 A | 31-12-1983 |
| WO 9710383 | A1 | 20-03-1997 | AT | E213516 T1 | 15-03-2002 |
| | | | CA | 2229499 A1 | 20-03-1997 |
| | | | DE | 69619397 T2 | 14-08-2002 |
| | | | EP | 0868570 A1 | 07-10-1998 |
| | | | JP | H11515060 A | 21-12-1999 |
| | | | KR | 19990044658 A | 25-06-1999 |
| | | | US | 5745244 A | 28-04-1998 |
| | | | WO | 9710383 A1 | 20-03-1997 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004277899 A
- JP 2003027396 A
- JP 2013108859 A